(19) Europäisches Patentamt
European Patent Office
Office européen des brevets

(11) EP 0 668 854 B1

(12) **FASCICULE DE BREVET EUROPEEN**

(45) Date de publication et mention de la délivrance du brevet: **13.10.1999 Bulletin 1999/41**

(21) Numéro de dépôt: **94900187.9**

(22) Date de dépôt: **10.11.1993**

(51) Int Cl.[6]: **C07C 229/16**, A61K 31/195, A61K 7/48, A61K 7/06, C07D 213/38

(86) Numéro de dépôt international: **PCT/FR93/01109**

(87) Numéro de publication internationale: **WO 94/11338 (26.05.1994 Gazette 1994/12)**

(54) **UTILISATION DE N-ARYLMETHYLENE ETHYLENEDIAMINETRIACETATES, N-ARYLMETHYLENE IMINODIACETATES OU N,N'-DIARYLMETHYLENE ETHYLENEDIAMINACETATES CONTRE LE STRESS OXYDANT**

VERWENDUNG VON N-ARYLMETHYLEN ETHYLENDIAMINTRIACETATE, N-ARYLMETHYLEN IMINODIACETAT ODER N,N'-DIARYLMETHYLEN ETHYLENDIAMINDIACETAT GEGEN OXIDATIVEN STRESS

USE OF N-ARYLMETHYLENE, ETHYLENEDIAMINETRIACETATES N-ARYLMETHYLENE IMINODIACETATES OR N,N'-DIARYLMETHYLENE ETHYLENEDIAMINACETATES FOR USE IN COMBATTING OXIDATIVE STRESS

(84) Etats contractants désignés:
**AT BE CH DE ES FR GB IT LI NL SE**

(30) Priorité: **13.11.1992 FR 9213707**
**23.06.1993 FR 9307641**

(43) Date de publication de la demande:
**30.08.1995 Bulletin 1995/35**

(73) Titulaire: **L'OREAL**
**75008 Paris (FR)**

(72) Inventeurs:
- **GALEY, Jean-Baptiste**
**F-75005 Paris (FR)**
- **DUMATS, Jacqueline**
**F-93240 Villepinte (FR)**

(74) Mandataire: **Casalonga, Axel et al**
**BUREAU D.A. CASALONGA - JOSSE**
**Morassistrasse 8**
**80469 München (DE)**

(56) Documents cités:

| | |
|---|---|
| **EP-A- 0 058 373** | **EP-A- 0 367 223** |
| **WO-A-90/00987** | **DE-A- 2 409 355** |
| **DE-A- 2 728 321** | **DE-A- 2 754 535** |
| **US-A- 3 886 284** | **US-A- 4 528 196** |

- **AMERICAN JOURNAL OF HEMATOLOGY vol. 24, no. 3 , Mars 1987 pages 277 - 284 BYOUNG-KOOK K. 'EFFECTIVENESS OF ORAL IRON CHELATORS ASSAYED IN THE RAT'**
- **INORGANICA CHIMICA ACTA vol. 138, no. 3 , 1987 pages 215 - 230 A. E. MARTELL ET AL 'DEVELOPMENT OF IRON CHELATORS FOR COOLEY'S ANEMIA'**
- **CHEMICAL ABSTRACTS, vol. 110, no. 13, 27 Mars 1989, Columbus, Ohio, US; abstract no. 110927, LI J ET AL. 'Specific sequestering agents for the actinides VI' page 344 ; & SHANDONG YIKE DAXUE XUEBAO (SYXBEE); 88; VOL.26 (2); PP.64-8 & CHEMICAL ABSTRACTS, vol. 24, no. 2, 25 Janvier 1960, Columbus, Ohio, US; abstract no. 110927, '12TH COLLECTIVE INDEX CHEMICAL SUBSTANCES:GLYCINE,N,N'-1,2 -ETHANEDIYLBIS[N-[(3-HYDROXY-4-4-METHO XYPH ENYL)METHYL]-' page 41612CS ;**
- **JOURNAL OF MEDICINAL CHEMISTRY vol. 29, no. 7 , Juillet 1986 , WASHINGTON US pages 1231 - 1237 C.G. PITT ET AL. 'ESTERS AND LACTONES OF PHENOLIC AMINO CARBOXYLIC ACIDS: PRODURUGS FOR IRON CHELATION'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

- CHEMICAL ABSTRACTS, Vol. 113, 1990, (Columbus, Ohio, US), page 27, abstrait no. 17571M, H. SUZUKI et al, "Cytocidal activity of a synthetic isoprenoid, N-celanesyl-N,N'-bis(3,4-dimethoxybenzyl)et hylenediamine, and its potentiation of antitumor drugs against multidrug-resistant and sensitive cells in vitro"
- CHEMICAL ABSTRACTS, Vol. 86, 1977, (Columbus, Ohio, US), page 512, abstrait no. 170981P, J. Strumillo, "Synthesis of 1,3,5-triazacycloheptane derivatives. I. Synthesis of alkyl- and arylethylenediamine derivatives"

## Description

La présente invention conceme des dérivés de N-arylméthylène éthylènediaminetriacétate, N-arylméthylène iminodiacétate ou N,N'-diarylméthylène éthylènediaminediacétate, utiles notamment pour protéger l'organisme contre le stress oxydant, leur procédé de préparation ainsi que les compositions pharmaceutiques et cosmétiques contenant de tels composés.

Dans le domaine de la santé et de la cosmétique, le concept du stress oxydant est connu, stress oxydant qui apparaît notamment dès qu'il existe un déséquilibre de la balance antioxydant-prooxydant. Ce déséquilibre se traduit notamment par des processus oxydatifs au sein des tissus vivants, non contrôlés, qui mettent en jeu des radicaux libres oxygénés et conduisent notamment à des dégâts oxydatifs sur les molécules et les macromolécules biologiques (Sies, H., In Oxidative Stress, Academic Press Inc. (London) Ltd, 1985).

Il est connu que des situations diverses provoquent, favorisent ou accompagnent le stress oxydant ou en sont la conséquence; il s'agit notamment de l'exposition aux rayons ultraviolets et aux rayonnements ionisants, du vieillissement, de l'inflammation, de la carcinogénèse, des situations d'ischémie reperfusion, de la toxicité et/ou du mode d'action de certains médicaments.

Lors de ce phénomène du stress oxydant, il est connu que du fer est libéré de ses sites de stockage usuels comme la ferritine et, libéré, peut participer à certaines réactions, et notamment aux réactions de Fenton (1) et Haber-Weiss (2) dont résulte la formation des radicaux hydroxyle, radicaux connus pour être responsables de nombreux dommages oxydatifs (Reif, D.W., Free Rad. Biol. Med. 12, 417-427, 1992).

L'oxygène est indispensable à la respiration des êtres vivants aérobies, mais peut être réduit en radical superoxyde $O_2^{\circ -}$ dans toutes les cellules aérobies. Ce radical peut subir une réaction de dismutation donnant naissance à du peroxyde d'hydrogène :

$$2\,O_2^{\circ -} + 2H^+ \rightarrow H_2O_2 + O_2$$

En présence de traces de fer, ce radical superoxyde peut également réduire l'ion $Fe^{3+}$ :

$$O_2^{\circ -} + Fe^{3+} \rightarrow Fe^{2+} + O_2$$

Les ions $Fe^{2+}$ ainsi produits peuvent donner lieu à la réaction de Fenton qui produit le radical hydroxyle :

$$H_2O_2 + Fe^{2+} \rightarrow OH^{\circ} + OH^- + Fe^{3+} \qquad (1)$$

La réaction d'Haber-Weiss produit elle aussi des radicaux hydroxyle :

$$H_2O_2 + O_2^{\circ -} \xrightarrow{Fe^{3+}} OH^{\circ} + OH^- + O_2 \qquad (2)$$

Le radical hydroxyle $OH^{\circ}$ peut provoquer des dégâts très importants dans l'organisme. Il est capable de casser des brins d'ADN et d'altérer le patrimoine génétique de la cellule vivante. Contrairement à $H_2O_2$ et au radical superoxyde $O_2^{\circ -}$, il est également capable de provoquer une peroxydation des acides gras insaturés. Il joue un rôle important dans le vieillissement de la peau.

Il est connu que la protection des tissus vivants contre les attaques du radical hydroxyle est difficile.

L'une des approches connue pour se protéger est d'utiliser des molécules, notamment le D-mannitol ou le DMSO (diméthylsulfoxyde), capables de piéger les radicaux hydroxyle. Néanmoins, le radical hydroxyle est une espèce si réactive qu'il faut utiliser des quantités très importantes de ces piégeurs, de manière à entrer en compétition avec toutes les molécules biologiques, cibles potentielles du radical hydroxyle (Halliwell, B., Free Rad. Res. Comms., 9(1), 1-32, 1990). L'utilisation de fortes quantités de ces piégeurs pose des problèmes de toxicité.

L'autre approche connue pour se protéger contre les radicaux hydroxyle est d'utiliser des chélateurs du fer, notamment la déféroxamine ou l'acide diéthylène triamine pentaacétique (DTPA) pour l'empêcher de participer aux réactions de Fenton et Haber-Weiss.

Cependant, si leurs constantes de complexation sont élevées, ces chélateurs peuvent être toxiques. C'est ainsi que le DTPA présente des effets secondaires importants, liés probablement pour une partie à la chélation de métaux tels que le calcium.

La déféroxamine présente une toxicité chronique supposée liée à sa capacité à chélater les métaux des sites actifs des métalloenzymes ou hémoprotéines comme l'hémoglobine.

Par ailleurs, des chélateurs puissants, qui peuvent complexer le fer, tels que l'EHPG (éthylène bis-o-hydroxy phényl glycine), sont également connus pour avoir des toxicités aiguës importantes.

Enfin, le HBED-DME c'est-à-dire l'ester diméthylique d'acide N,N'-bis(2-hydroxybenzyl)-éthylène diamine diacétique, est décrit en tant que chélateur du fer exceptionnellement efficace dans le brevet US 4.528.196 et dans la publication "American Journal of Hematology", Vol. 24, No 3, mars 1987. Le HBED, c'est-à-dire l'acide N,N'-bis(2-hydroxybenzyl) éthylène diamine diacétique, est décrit dans Chemical Abstracts, Vol. 110, 1989, 110 927e, en tant qu'agent séquestrant pour actinides. Ces composés présentent également des risques de toxicité car ils forment des complexes très stables, tous les sites de coordination du fer étant occupés du fait de la présence des groupes OH en position 2.

La demanderesse a découvert que des dérivés de N-arylméthylène éthylènediaminetriacétate, N-arylméthylène iminodiacétate ou N,N'-diarylméthylène éthylènediaminediacétate, étaient particulièrement efficaces pour protéger l'organisme contre le stress oxydant.

Sans que cette explication soit limitative, il semble que cet effet soit dû à la faculté de ces dérivés non seulement de former avec le fer des complexes, mais encore de piéger les radicaux hydroxyle, de façon quasi stoechiométrique, avant qu'ils ne puissent attaquer d'autres molécules.

En effet, les composés selon l'invention forment des complexes avec l'ion $Fe^{2+}$ et ces complexes sont capables de décomposer le peroxyde d'hydrogène sans libérer de radicaux hydroxyle. Ces radicaux sont en effet formés mais aussitôt piégés par un processus d'hydroxylation intramoléculaire, ce qui permet de n'utiliser que de très faibles concentrations des molécules selon l'invention. Ce dernier point constitue un avantage par rapport aux autres piégeurs de radicaux hydroxyle déjà cités qui nécessitent d'être utilisés en très large excès.

Un autre avantage des composés selon l'invention, est que ceux-ci forment avec le fer des complexes dont les constantes d'association sont beaucoup plus faibles que celles des composés cités plus haut comme la déféroxamine ou l'HBED. Les risques toxicologiques sont donc diminués.

Enfin, les produits d'hydroxylation intramoléculaire des complexes ferreux des molécules selon l'invention ont une grande affinité pour le fer et forment avec celui-ci des complexes capables de l'empêcher de catalyser la formation d'autres radicaux hydroxyle.

L'invention a pour objet l'utilisation pour la protection de l'organisme contre le stress oxydant, et notamment comme piégeurs de radicaux libres hydroxyle et chélateurs du fer, de dérivés de N-arylméthylène éthylènediaminetriacétate, N-arylméthylène iminodiacétate ou N,N'-diarylméthylène éthylènediaminediacétate.

Un autre objet est constitué par les compositions cosmétiques et pharmaceutiques les mettant en oeuvre.

L'invention a également pour objet les composés nouveaux de la famille des N-arylméthylène éthylènediaminetriacétate, N-arylméthylène iminodiacétate ou N,N'-diarylméthylène éthylènediaminediacétate et leur préparation.

D'autres objets de l'invention apparaîtront à la lecture de la description et des exemples qui suivent.

Les composés utilisés conformément à l'invention pour la protection contre le stress oxydant, notamment par piégeage de radicaux libres hydroxyle et complexation du fer, sont les composés de formule (I) :

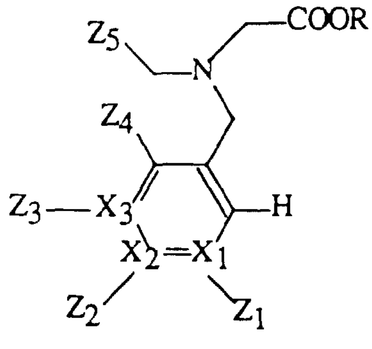

(I)

dans laquelle :

$Z_1$, $Z_2$, $Z_3$, indépendamment l'un de l'autre, représentent $NO_2$, COOH, $CF_3$, un atome d'halogène ou un groupement $R_1$, $OR_1$, $SR_1$ ou $NR_1R_2$,

$Z_4$ représente un groupement $R_1$;

où R, $R_1$ et $R_2$, indépendamment l'un de l'autre, représentent H ou un groupement alkyle linéaire ou ramifié, en

$C_1$ à $C_8$,
$X_1$, $X_2$, $X_3$ représentent :

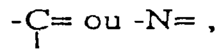

à condition que
si $X_1$=N, alors $X_2$=$X_3$=C et il n'y a pas de substituant $Z_1$ sur $X_1$ ,
si $X_2$=N, alors $X_1$=$X_3$=C et il n'y a pas de substituant $Z_2$ sur $X_2$ ,
si $X_3$=N, alors $X_2$=$X_1$=C et il n'y a pas de substituant $Z_3$ sur $X_3$ , c'est-à-dire qu'il s'agit d'un noyau de pyridine;
si $X_1$, $X_2$ et $X_3$ représentent tous trois C, il s'agit alors d'un noyau de benzène ;
$Z_5$ représente :

le groupement :

$$-COOR \quad (a)$$

ou le groupement :

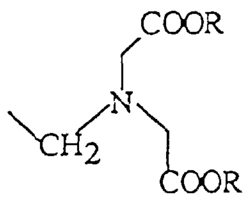

(b)

ou le groupement :

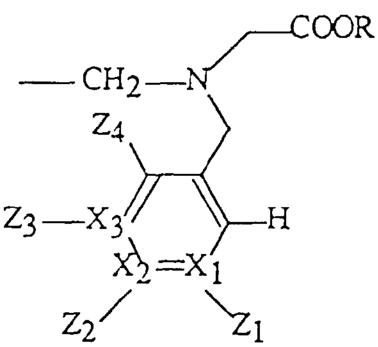

(c)

dans lesquels $Z_1$, $Z_2$, $Z_3$, $Z_4$, $X_1$, $X_2$, $X_3$, R, $R_1$ et $R_2$ ont les mêmes significations que ci-dessus;
ainsi que leurs sels et leurs complexes métalliques.

Les groupements alkyle, linéaires ou ramifiés en $C_1$-$C_8$, sont de préférence des groupements alkyle en $C_1$-$C_4$, tels que méthyle, éthyle, isopropyle, tert-butyle.

Comme sels, on peut citer les sels d'addition avec un acide minéral comme les acides $H_2SO_4$, HCl, $HNO_3$ ou $H_3PO_4$, par exemple, et les sels d'addition avec une base minérale comme NaOH ou KOH.

Comme complexes métalliques, on peut citer les complexes formés par addition de $ZnCl_2$ ou $CaCl_2$, par exemple.

Les composés nouveaux sont les composés de formule (I), dans laquelle $Z_1$, $Z_2$, $Z_3$, $Z_4$, $Z_5$, $X_1$, $X_2$, $X_3$, R, $R_1$, $R_2$ ont les significations indiquées précédemment, ainsi que leurs sels et complexes métalliques, à l'exclusion des produits ci-dessous qui sont déjà connus :

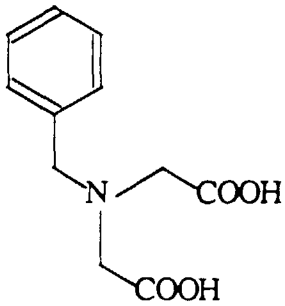

acide benzyl imino-diacétique

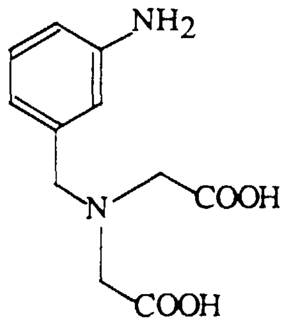

acide 3-aminobenzyl imino-diacétique

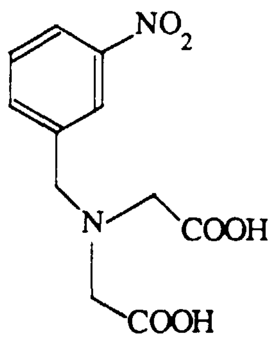

acide 3-nitrobenzyl iminodiacétique

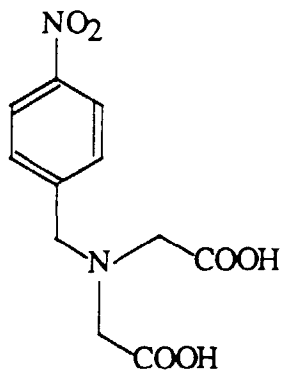

acide 4-nitrobenzyl iminodiacétique

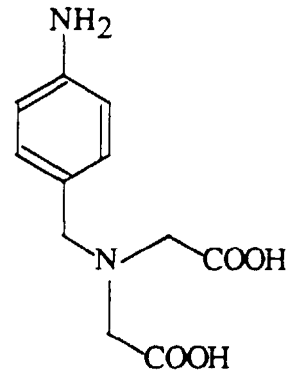

acide 4-aminobenzyl iminodiacétique

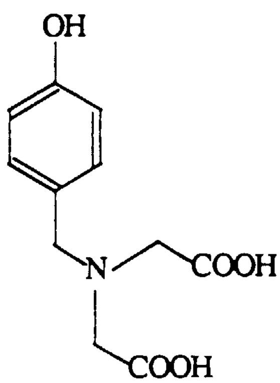

acide 4-hydroxybenzyl iminodiacétique

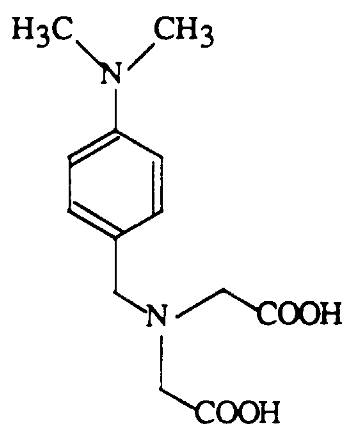

acide 4-diméthylamino-benzyl iminodiacétique

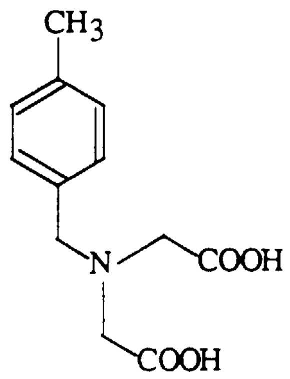

acide 4-méthylbenzyl iminodiacétique

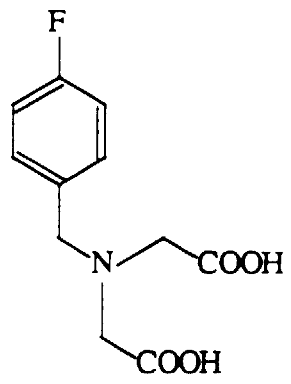

acide 4-fluorobenzyl iminodiacétique

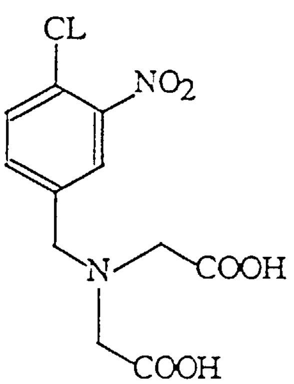

acide 3-nitro-4-chloro-benzyl iminodiacétique

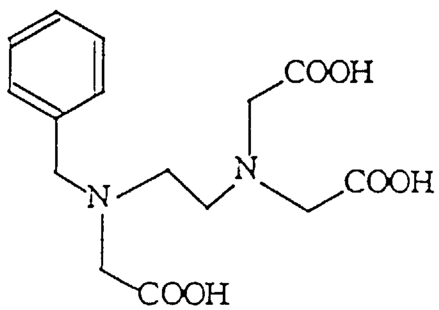

acide benzyl éthylènediamine triacétique

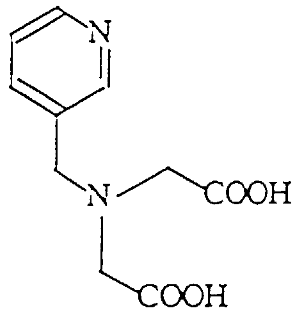

acide 3-pyridylméthylène iminodiacétique

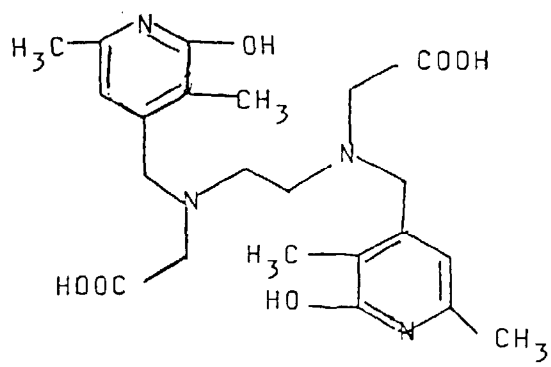

acide N,N'-bis(3-hydroxy-2,5-diméthyl-4-pyridylméthylène) éthylène diamine diacétique.

et sous réserve que :

lorsque $Z_5$ désigne le groupement (c) dans lequel $X_1$, $X_2$, $X_3$ représentent C et $Z_4$ désigne H,
l'un au moins de $Z_1$ et $Z_3$ est différent de H lorsque $Z_2$ désigne H, Cl, $NO_2$ ou $CH_3$ et
$Z_3$ n'est pas H lorsque $Z_1$ désigne OH et $Z_2$ désigne $OCH_3$.

Les composés définis par la formule (I) sont utilisés comme médicaments, notamment pour protéger l'organisme des effets néfastes des radicaux libres dus en particulier au stress oxydant. Ils sont particulièrement utilisables pour traiter les situations de stress oxydant liées à des états pathologiques chez l'homme ou l'animal, comme les cancers, les états inflammatoires, l'ischémie reperfusion, les surcharges en fer, les maladies dégénératives du système nerveux, ou encore pour traiter les effets liés à l'exposition aux rayonnements ionisants ou à l'utilisation de certains médicaments connus pour générer des radicaux libres, notamment des médicaments anticancéreux comme l'adriamycine, etc.

Les composés conformes à l'invention peuvent également être utilisés pour traiter les situations de stress oxydant liées à des états non pathologiques, comme ceux résultant de l'exposition au soleil ou dus au vieillissement. Ils sont utilisés dans ce cas-là par voie topique pour leur effet cosmétique sur la peau ou les cheveux.

Les compositions cosmétiques et pharmaceutiques mettant en oeuvre les composés de formule (I) comportent un composé de formule (I) ou l'un de ses sels ou complexes métalliques, dans un milieu cosmétiquement ou pharmaceutiquement acceptable.

Ces compositions contiennent les composés de formule (I) dans des proportions de 0,001 à 10% en poids.

Les compositions cosmétiques et pharmaceutiques peuvent se présenter sous des formes diverses habituellement utilisées dans ce domaine, et en particulier sous forme d'onguent, de crème, de pommade, de comprimé, de suspension buvable, d'injection ou de gel pour les compositions pharmaceutiques et sous forme de gel, de spray, de lotion, d'émulsion ou de dispersion vésiculaire pour les compositions cosmétiques.
Lorsque les composés de formule (I) sont utilisés dans le cadre d'un traitement pharmaceutique, les formes d'administration peuvent adopter la voie orale, topique ou parentérale, le support pharmaceutiquement acceptable dépendant de la forme d'administration choisie. La posologie est généralement comprise entre 1 et 100 mg/kg/jour.
Le milieu cosmétiquement ou pharmaceutiquement acceptable est un milieu usuel dans le domaine cosmétique ou pharmaceutique.
Les composés de formule (I) peuvent, selon. une forme de réalisation préférée, être utilisés avec au moins un autre agent actif (ou un autre agent anti-radicaux libres). Ces agents peuvent être choisis plus particulièrement parmi :

- les antilipoperoxydants comme la vitamine E, le trolox, le BHT (butylhydroxytoluène),
- un réducteur biologique comme le glutathion réduit et ses dérivés, la vitamine C et ses dérivés,
- un capteur d'oxygène singulet comme un caroténoïde tel que le β-carotène,
- un système capable de décomposer le peroxyde d'hydrogène tel que des enzymes comme la catalase ou les peroxydases en présence de leur co-substrat,
- un système de protection contre l'anion superoxyde comme les SOD ou des analogues tels que le complexe Mn-desferal ou le di-isopropyl salicylate de cuivre,
- un système capable de décomposer les hydroperoxydes organiques comme la glutathion peroxydase ou des systèmes à base de sélénium,
- les agents anti-inflammatoires,
- les filtres UV,
- les promoteurs de pénétration,

et les associations de ces composés.
Les composés de formule (I) (composant (A)) et les agents actifs ou agents anti-radicaux libres (composant (B)) définis ci-dessus, peuvent être mis en oeuvre dans la même composition ou être appliqués séparément, de façon décalée ou non, dans le temps, à l'aide d'une composition cosmétique ou pharmaceutique les contenant.
Par ailleurs, la demanderesse a constaté que les composés (I) selon l'invention sont utilisables comme antioxydants pour conserver les compositions les contenant.
La présente invention concerne également le procédé de préparation des composés (I) de l'invention.
Le procédé varie selon la nature de $Z_5$.
Lorsque $Z_5$ = - COOR (a), selon le procédé (A), l'aldéhyde de formule (II) (1 mole) :

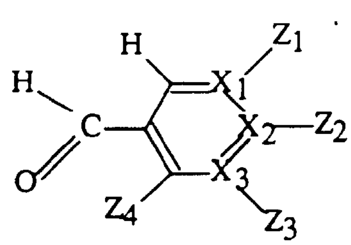

(II)

où $X_1$, $X_2$, $X_3$, $Z_1$, $Z_2$, $Z_3$ et $Z_4$ ont les significations déjà indiquées, est mis en présence de l'ester éthylique de la glycine (1 mole) pour obtenir l'imine correspondante.
Lorsque $Z_5$ désigne

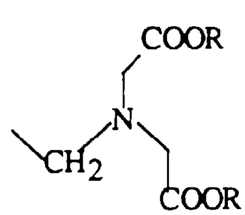

(b)

selon le procédé (B), l'aldéhyde de formule (II) (1 mole) est mis en présence de N-acétyl éthylènediamine (1 mole) pour obtenir l'imine correspondante.

Lorsque $Z_5$ désigne

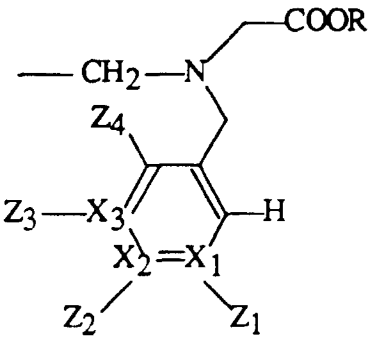

(c)

selon le procédé (C), l'aldéhyde de formule (II) (2 moles) est mis en présence d'éthylènediamine (1 mole) pour obtenir la diimine correspondante.

Dans chacun des trois procédés, l'imine ou la diimine obtenue, isolée ou non, est mise en présence de borohydrure de sodium ou est réduite par hydrogénation catalytique, pour donner naissance à l'amine ou à la diamine correspondante.

Selon le procédé (B), la diamine obtenue est alors traitée par l'acide chlorhydrique pour hydrolyser la fonction acétyle.

Selon le procédé (A), l'amine obtenue est traitée par la soude pour saponifier l'ester éthylique.

Dans une étape ultérieure, lamine ou la diamine obtenue par les procédés (A), (B) ou (C), est traitée en milieu basique, par exemple en présence d'hydroxyde de sodium, avec l'acide bromoacétique ou l'un de ses esters, en présence de monohydrogénocarbonate de sodium. On récupère alors le produit de formule (I) correspondant. Ces procédés sont schématisés comme suit :

## SCHEMA DE SYNTHESE GENERAL

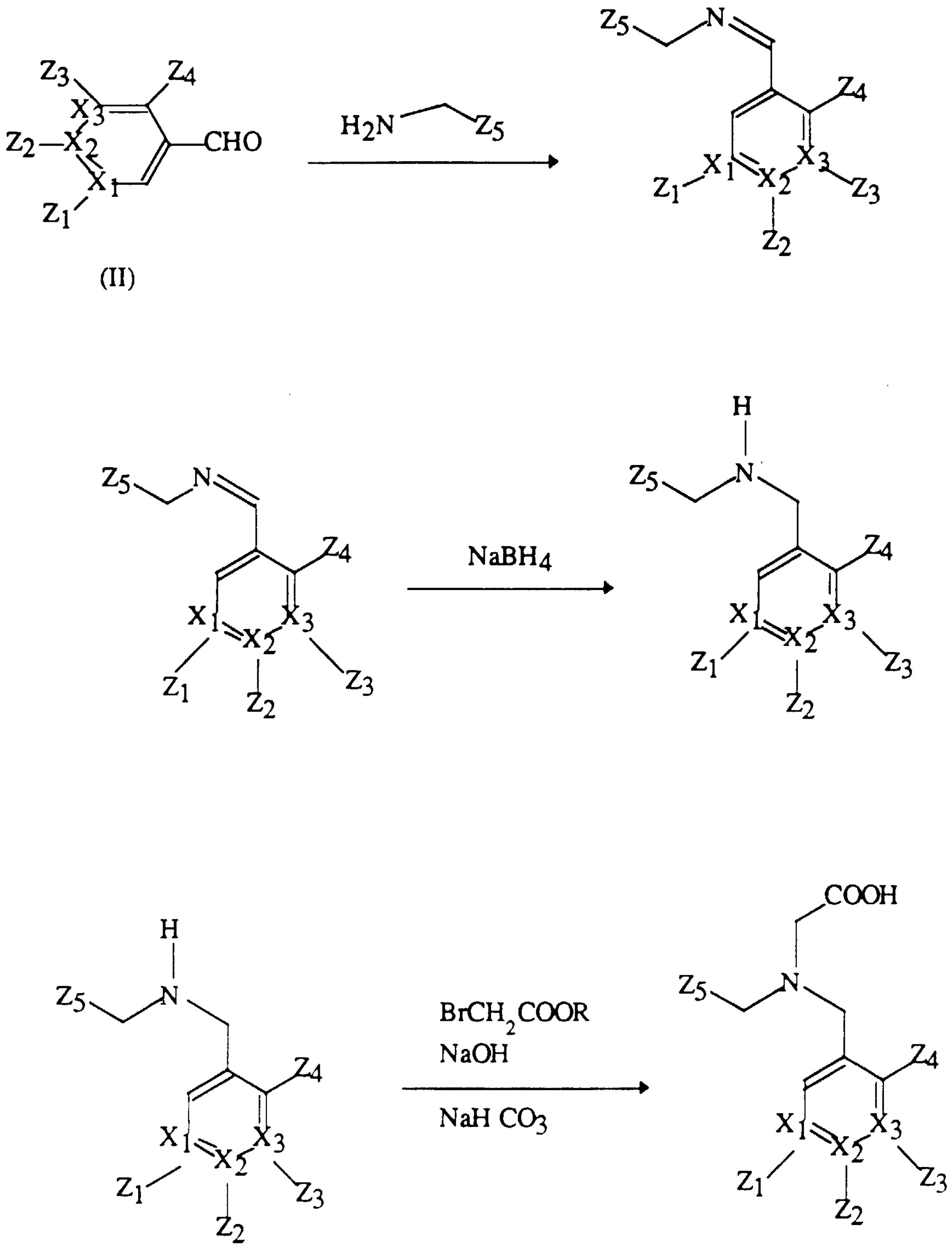


(II)

Selon la nature de $Z_1$, $Z_2$ et $Z_3$, une étape de protection/déprotection peut être nécessaire, notamment dans le cas où l'un de ces substituants est $NH_2$. Cette étape supplémentaire est faite selon les techniques usuelles de la chimie organique, de même que l'estérification finale des composés selon la formule (I) pour lesquels R est H, si on utilise l'acide bromo-acétique libre.

D'autres caractéristiques et avantages de l'invention apparaîtront à la lecture des exemples ci-après.

EXEMPLES DE PREPARATION

Exemple 1

acide 3,4,5-triméthoxybenzyl iminodiacétique

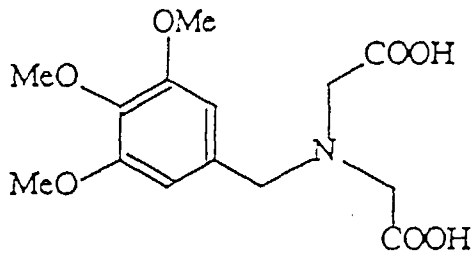

Exemple 2

acide benzyl iminodiacétique

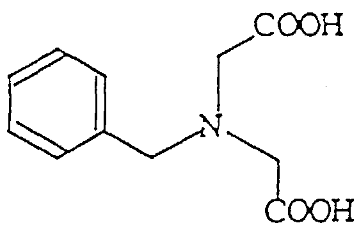

Exemple 3

acide 3-hydroxybenzyl iminodiacétique

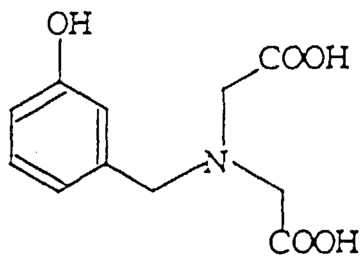

Exemple 4

acide N-benzyl éthylène diaminetriacétique

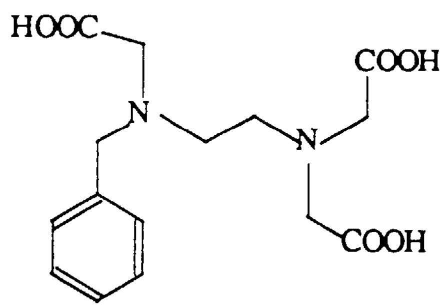

| Exemple | point de fusion | RMN $^{13}$C ou $1_H$ et SM |
|---|---|---|
| N° 1 | 206-208°C | conformes |
| N° 2 | 200°C | conformes |
| N° 3 | 222°C | conformes |
| N° 4 | 250°C | conformes |

Exemples 5 à 18

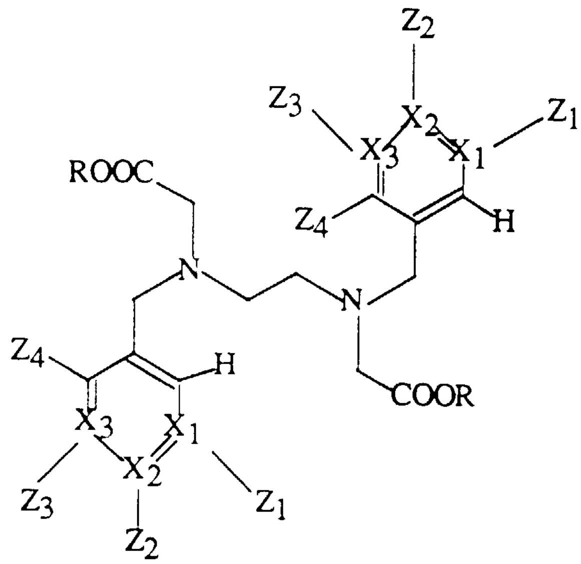

| Exemple | $X_1$ | $X_2$ | $X_3$ | $Z_1$ | $Z_2$ | $Z_3$ | $Z_4$ | R | PF °C | RMN $^{13}C$ ou $^{1}H$ et SM |
|---|---|---|---|---|---|---|---|---|---|---|
| 5 | C | C | C | H | H | H | Me | H | 198 | Conformes |
| 6 | C | C | C | H | H | OH | H | H | 175 | " |
| 7 | C | C | C | H | OMe | OH | H | H | > 260 | " |
| 8 | C | C | C | OMe | OMe | OMe | H | H | 180 | " |
| 9 | C | C | C | H | OH | H | H | H | 255 | " |
| 10 | C | C | C | H | H | H | H | H | > 260 | " |
| 11 | C | C | C | OMe | H | H | H | H | 174 | " |
| 12 | C | C | C | OMe | H | OMe | H | H | 170 | " |
| 13 | C | C | C | OH | $NO_2$ | H | H | H | 220 | " |
| 14 | C | C | C | Cl | H | H | H | H | > 260 | " |
| 15 | C | C | C | H | OMe | Me | Me | H | > 260 | " |
| 16 | C | C | C | H | H | H | H | isoPr | 50 | " |
| 17 | C | C | C | OMe | OMe | OMe | H | Me | 80 | " |
| 18 | N | C | C | - | H | H | H | H | 218 | " |

**MODE OPERATOIRE GENERAL**

Première étape

40 mmoles du benzaldéhyde de formule (II) de départ sont solubilisées dans 30 ml de méthanol. On additionne 40 mmoles de l'amine (20 mmoles dans le cas de l'éthylènediamine), puis le mélange est chauffé 30 minutes à 50°C.
Le précipité obtenu est filtré et lavé à l'éther éthylique. On obtient un produit blanc.

Deuxième étape

17 mmoles de l'imine ou de la diimine sont mises en suspension dans 100 ml d'éthanol absolu. On additionne peu à peu 1 équivalent de borohydrure de sodium et on agite le mélange 1 heure à température ambiante.
Après évaporation, on ajoute 20 ml d'eau au résidu et on ramène le pH à 8 par addition d'acide chlorhydrique.
Le précipité est filtré, lavé à l'eau puis séché.
Le produit est recristallisé dans un mélange eau/éthanol. On obtient un produit blanc.
Dans le cas de la synthèse du composé n°9, le produit est solubilisé dans 30 ml d'éthanol, puis 4 ml d'une

solution d'éthanol chlorhydrique à 8 moles/l sont ajoutés. Le précipité obtenu est filtré puis lavé à l'éther. On obtient le dichlorhydrate de la diamine sous forme d'un produit blanc.

Troisième étape

Selon le procédé (C) (exemples n°5 à 18), 10 mmoles de la diamine sont solubilisées dans 15 ml d'eau contenant 10 mmoles d'hydroxyde de sodium (30 mmoles si on part du dichlorhydrate).

20 mmoles d'acide bromoacétique sont solubilisées dans 25 ml d'eau à 0°C contenant 20 mmoles de monohydrogénocarbonate de sodium.

Les deux solutions sont mélangées et chauffées à 40°C pendant 6 heures en maintenant le pH vers 12 par addition de soude à 30%.

Après une nuit au repos à température ambiante, le mélange est acidifié par de l'acide chlorhydrique concentré jusqu'à pH 4-5.

La solution est concentrée sous vide et le précipité obtenu est filtré puis recristallisé dans un mélange eau/éthanol. On récupère une poudre blanche.

Selon le procédé (A) (exemples n° 1, 2, et 3), 10 mmoles d'amine dans 20 ml d'eau sont préalablement agitées 30 minutes à température ambiante en présence de 10 mmoles de NaOH. Le mélange brut obtenu est alors traité par 10 mmoles d'acide bromoacétique solubilisées dans 25 ml d'eau contenant 10 mmoles de $NaHCO_3$, comme indiqué ci-dessus pour le procédé (C).

Selon le procédé (B) (exemple n° 4), 10 mmoles de diamine dans 20 ml d'HCl 4N sont portées au reflux pendant 24 heures. Le mélange brut obtenu est alors ramené à pH basique et traité par 30 mmoles d'acide bromoacétique solubilisées dans 30 ml d'eau contenant 30 mmoles de $NaHCO_3$ pour obtenir le dérivé N-benzyléthylène diamine correspondant, comme indiqué ci-dessus pour le procédé (C).

## EXEMPLES DE FORMULATION COSMETIQUE

### EXEMPLE A

On prépare l'émulsion suivante selon les techniques classiques en utilisant les constituants ci-dessous.

- Composé de l'exemple 1 0,1 g
- Polyéthylèneglycol oxyéthyléné à 50 moles d'oxyde d'éthylène 3 g
- Monodiglycérylstéarate 3 g
- Huile de vaseline 24 g
- Alcool cétylique 5 g
- Eau qsp 100 g

On obtient une émulsion blanche destinée à être appliquée topiquement sur la zone de la peau à protéger.

### EXEMPLE B

On prépare l'émulsion suivante selon les techniques classiques en utilisant les constituants ci-dessous.

- Composé de l'exemple 2 0,02 g
- Octylpalmitate 10 g
- Glycérylisostéarate 4 g
- Huile de vaseline 24 g
- Vitamine E 1 g
- Glycérol 3 g
- Eau qsp 100 g

On obtient une émulsion blanche destinée à être appliquée topiquement sur la zone de la peau à protéger.

### EXEMPLE C

On prépare la formulation suivante selon les techniques classiques en utilisant les constituants ci-dessous.

- Composé de l'exemple 2 0,02 g

- Huile de jojoba 13 g
- Parahydroxybenzoates de méthyle et d'isopropyle 0,05 g
- Sorbate de potassium 0,3 g
- Cyclopentadiméthylsiloxane 10 g
- Alcool stéarylique 1 g
- Acide stéarique 4 g
- Stéarate de polyéthylèneglycol 3 g
- Vitamine E 1 g
- Glycérol 3 g
- Eau qsp 100 g

On obtient une émulsion blanche destinée à être appliquée topiquement sur la zone de la peau à protéger.

EXEMPLE D

On prépare l'émulsion suivante selon les techniques classiques en utilisant les constituants ci-dessous.

- Composé de l'exemple 6 0,1 g
- Polyéthylèneglycol oxyéthyléné à 50 moles d'oxyde d'éthylène 3 g
- Monodiglycérylstéarate 3 g
- Huile de vaseline 24 g
- Alcool cétylique 5 g
- Eau qsp 100 g

On obtient une émulsion blanche destinée à être appliquée topiquement sur la zone de la peau à protéger.

EXEMPLE E

On prépare l'émulsion suivante selon les techniques classiques en utilisant les constituants ci-dessous.

- Composé de l'exemple 7 0,02 g
- Octylpalmitate 10 g
- Glycérylisostéarate 4 g
- Huile de vaseline 24 g
- Vitamine E 1 g
- Glycérol 3 g
- Eau qsp 100 g

On obtient une émulsion blanche destinée à être appliquée topiquement sur la zone de la peau à protéger.

EXEMPLE F

On prépare la formulation suivante selon les techniques classiques en utilisant les constituants ci-dessous.

- Composé de l'exemple 6 0,02 g
- Huile de jojoba 13 g
- Parahydroxybenzoates de méthyle et d'isopropyle 0,05 g
- Sorbate de potassium 0,3 g
- Cyclopentadiméthylsiloxane 10 g
- Alcool stéarylique 1 g
- Acide stéarique 4 g
- Stéarate de polyéthylèneglycol 3 g
- Vitamine E 1 g
- Glycérol 3 g
- Eau qsp 100 g

On obtient une émulsion blanche destinée à être appliquée topiquement sur la zone de la peau à protéger.

**EXEMPLES DE FORMULATION PHARMACEUTIQUE**

**G - VOIE ORALE**

1) *Comprimé*

- Composé de l'exemple 2 0,001 g
- Amidon 0,114 g
- Phosphate bicalcique 0,020 g
- Lactose 0,060 g
- Stéarate de magnésium 0,005 g

Après compactage, on obtient un comprimé de 0,2 g.

2) *Suspension buvable*

- Composé de l'exemple 2 0,001 g
- Glycérol 0,500 g
- Sorbitol à 70% 0,500 g
- Saccharinate de sodium 0,010 g
- Parahydroxybenzoate de méthyle 0,040 g
- Arôme qs
- Eau purifiée qsp 5 ml

3) *Comprimé*

- Composé de l'exemple 6 0,001 g
- Amidon 0,114 g
- Phosphate bicalcique 0,020 g
- Lactose 0,060 g
- Stéarate de magnésium 0,005 g

Après compactage, on obtient un comprimé de 0,2 g.

4) *Suspension buvable*

- Composé de l'exemple 8 0,001 g
- Glycérol 0,500 g
- Sorbitol à 70% 0,500 g
- Saccharinate de sodium 0,010 g
- Parahydroxybenzoate de méthyle 0,040 g
- Arôme qs
- Eau purifiée qsp 5 ml

**H - ADMINISTRATION PAR INJECTION**

*Ampoule injectable de 3 ml*

- Composé de l'exemple 2 0,002 g
- Hydroxyde de sodium 0,0007 g
- Eau pour préparation injectable qsp 3 ml

*Ampoule injectable de 3 ml*

- Composé de l'exemple 9 0,002 g
- Hydroxyde de sodium 0,0007 g
- Eau pour préparation injectable qsp 3 ml

**Revendications**

1. Utilisation d'un composé de formule (I) :

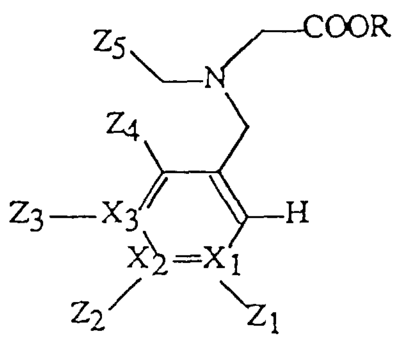

(I)

dans laquelle :

$Z_1$, $Z_2$, $Z_3$, indépendamment l'un de l'autre, représentent $NO_2$, COOH, $CF_3$, un atome d'halogène ou un groupement $R_1$, $OR_1$, $SR_1$ ou $NR_1R_2$,
$Z_4$ représente un groupement $R_1$;
où R, $R_1$ et $R_2$, indépendamment l'un de l'autre, représentent H ou un groupement alkyle linéaire ou ramifié, en $C_1$ à $C_8$,
$X_1$, $X_2$, $X_3$ représentent :

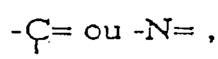

à condition que
si $X_1$=N, alors $X_2$=$X_3$=C et il n'y a pas de substituant $Z_1$ sur $X_1$ ,
si $X_2$=N, alors $X_1$=$X_3$=C et il n'y a pas de substituant $Z_2$ sur $X_2$ ,
si $X_3$=N, alors $X_2$=$X_1$=C et il n'y a pas de substituant $Z_3$ sur $X_3$ , c'est-à-dire qu'il s'agit d'un noyau de pyridine;
si $X_1$, $X_2$ et $X_3$ représentent tous trois C, il s'agit alors d'un noyau de benzène ;
$Z_5$ représente :

le groupement :

-COOR (a)

ou le groupement :

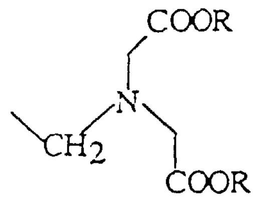

(b)

ou le groupement :

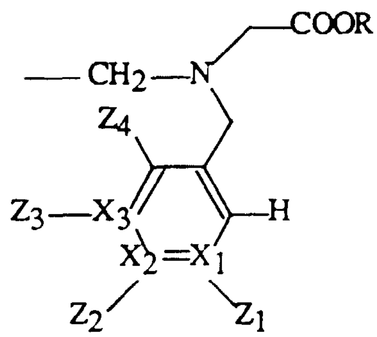

(c)

dans lesquels $Z_1$, $Z_2$, $Z_3$, $Z_4$, $X_1$, $X_2$, $X_3$, R, $R_1$ et $R_2$ ont les mêmes significations que ci-dessus;
ou de ses sels et complexes métalliques, pour le traitement cosmétique de l'organisme, en vue de le protéger contre le stress oxydant.

**2.** Composition cosmétique, caractérisée par le fait qu'elle contient au moins un composé de formule (I) tel que défini dans la revendication 1, dans un milieu cosmétiquement acceptable.

**3.** Composition selon la revendication 2, caractérisée par le fait qu'elle contient 0,001 à 10% en poids d'au moins un composé de formule (I) tel que défini dans la revendication 1.

**4.** Composition destinée au traitement cosmétique des effets du stress oxydant ou à la protection contre ces effets, caractérisée par le fait qu'elle comprend deux composants (A) et (B) :

le composant (A) étant constitué par le composé de formule (I) tel que défini dans la revendication 1,
le composant (B) étant choisi parmi les antilipoperoxydants, les réducteurs biologiques, les capteurs d'oxygène singulet, les systèmes capables de décomposer le peroxyde d'hydrogène, les systèmes de protection contre l'anion superoxyde, les systèmes capables de décomposer les hydroperoxydes organiques, les filtres UV, les promoteurs de pénétration ou leurs associations, et étant présent dans la même composition que le composant (A) ou destiné à être utilisé de façon séparée, décalée ou non dans le temps.

**5.** Composition selon la revendication 4, caractérisée par le fait que l'agent antilipoperoxydant est choisi parmi la vitamine E, le trolox, le BHT; le réducteur biologique est choisi parmi le glutathion réduit ou la vitamine C; le capteur d'oxygène singulet est un caroténoïde; le système capable de décomposer le peroxyde d'hydrogène est une enzyme catalase ou peroxydase; le système de protection contre l'anion superoxyde est constitué par les SOD, le complexe Mn-desferal, le di-isopropyl salicylate de cuivre; le système capable de décomposer les hydroperoxydes organiques est la glutathion peroxydase ou un système à base de sélénium.

**6.** Composé de formule (I) :

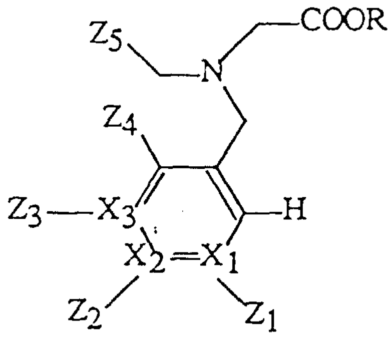

(I)

dans laquelle :

$Z_1$, $Z_2$, $Z_3$, indépendamment l'un de l'autre, représentent $NO_2$, COOH, $CF_3$, un atome d'halogène ou un groupement $R_1$, $OR_1$, $SR_1$ ou $NR_1R_2$,
$Z_4$ représente un groupement $R_1$;
où R, $R_1$ et $R_2$, indépendamment l'un de l'autre, représentent H ou un groupement alkyle linéaire ou ramifié, en $C_1$ à $C_8$,
$X_1$, $X_2$, $X_3$ représentent :

$$-\underset{|}{C}= \text{ ou } -N= \, ,$$

à condition que
si $X_1$=N, alors $X_2$=$X_3$=C et il n'y a pas de substituant $Z_1$ sur $X_1$ ,
si $X_2$=N, alors $X_1$=$X_3$=C et il n'y a pas de substituant $Z_2$ sur $X_2$ ,
si $X_3$=N, alors $X_2$=$X_1$=C et il n'y a pas de substituant $Z_3$ sur $X_3$ , c'est-à-dire qu'il s'agit d'un noyau de pyridine;
si $X_1$, $X_2$ et $X_3$ représentent tous trois C, il s'agit alors d'un noyau de benzène ;
$Z_5$ représente :

le groupement :

-COOR (a)

ou le groupement :

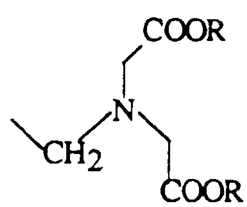

(b)

ou le groupement :

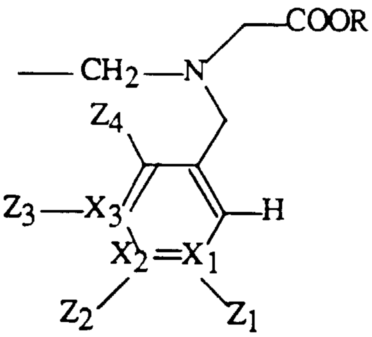

(c)

dans lesquels $Z_1$, $Z_2$, $Z_3$, $Z_4$, $X_1$, $X_2$, $X_3$, R, $R_1$ et $R_2$ ont les mêmes significations que ci-dessus; ou ses sels ou complexes métalliques, pour son utilisation dans une méthode de traitement thérapeutique du corps humain ou animal.

**7.** Composé répondant à la formule (I) tel que défini dans la revendication 6, pour son utilisation dans la protection de l'organisme humain contre le stress oxydant.

**8.** Composition pharmaceutique, caractérisée par le fait qu'elle contient dans un milieu pharmaceutiquement acceptable, au moins un composé de formule (I) tel que défini dans la revendication 6.

**9.** Composition pharmaceutique selon la revendication 8, caractérisée par le fait qu'elle contient 0,001 à 10% en poids d'au moins un composé de formule (I) tel que défini dans la revendication 6.

**10.** Composition destinée au traitement thérapeutique des effets du stress oxydant ou à la protection contre ces effets, caractérisée par le fait qu'elle comprend deux composants (A) et (B) :

le composant (A) étant constitué par le composé de formule (I) tel que défini dans la revendication 6,
le composant (B) étant choisi parmi les antilipoperoxydants, les réducteurs biologiques, les capteurs d'oxygène singulet, les systèmes capables de décomposer le peroxyde d'hydrogène, les systèmes de protection contre l'anion superoxyde, les systèmes capables de décomposer les hydroperoxydes organiques, les agents antiinflammatoires, les promoteurs de pénétration ou leurs associations, et étant présent dans la même composition que le composant (A) ou destiné à être utilisé de façon séparée, décalée ou non dans le temps.

**11.** Composé de formule (I) :

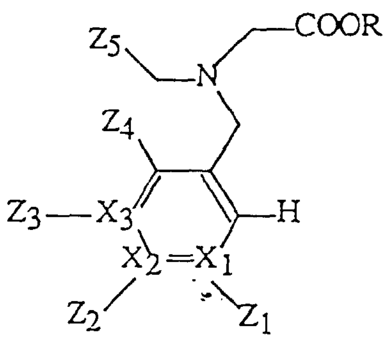

(I)

dans laquelle :

$Z_1$, $Z_2$, $Z_3$, indépendamment l'un de l'autre, représentent $NO_2$, COOH, $CF_3$, un atome d'halogène ou un groupement $R_1$, $OR_1$, $SR_1$ ou $NR_1R_2$,
$Z_4$ représente un groupement $R_1$;
où R, $R_1$ et $R_2$, indépendamment l'un de l'autre, représentent H ou un groupement alkyle linéaire ou ramifié,

en $C_1$ à $C_8$,
$X_1$, $X_2$, $X_3$ représentent :

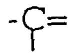 ou $-N=$ ,

à condition que
si $X_1$=N, alors $X_2$=$X_3$=C et il n'y a pas de substituant $Z_1$ sur $X_1$ ,
si $X_2$=N, alors $X_1$=$X_3$=C et il n'y a pas de substituant $Z_2$ sur $X_2$ ,
si $X_3$=N, alors $X_2$=$X_1$=C et il n'y a pas de substituant $Z_3$ sur $X_3$ , c'est-à-dire qu'il s'agit d'un noyau de pyridine;
si $X_1$, $X_2$ et $X_3$ représentent tous trois C, il s'agit alors d'un noyau de benzène ;
$Z_5$ représente :

le groupement :

$$-COOR \qquad (a)$$

ou le groupement :

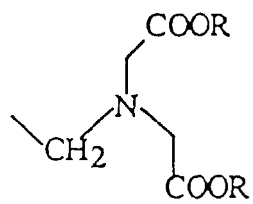

(b)

ou le groupement :

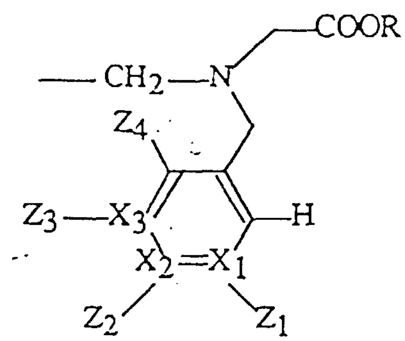

(c)

dans lesquels $Z_1$, $Z_2$, $Z_3$, $Z_4$, $X_1$, $X_2$, $X_3$, R, $R_1$ et $R_2$ ont les mêmes significations que ci-dessus;
à l'exclusion des composés suivants : acide benzyl iminodiacétique, acide 3-aminobenzyl iminodiacétique, acide 3-nitrobenzyl iminodiacétique, acide 4-nitrobenzyl iminodiacétique, acide 4-aminobenzyl iminodiacétique, acide 4-hydroxybenzyl iminodiacétique, acide 4-diméthylaminobenzyl iminodiacétique, acide 4-méthylbenzyl iminodiacétique, acide 4-fluorobenzyl iminodiacétique, acide 3-nitro-4-chlorobenzyl iminodiacétique, acide benzyl éthylènediaminetriacétique, acide 3-pyridylméthylène iminodiacétique, acide, N,N'-bis(3-hydroxy-2,5-diméthyl-4-pyridylméthylène)éthylène diamine diacétique et sous réserve que, lorsque $Z_5$ désigne le groupement (c), dans lequel $X_1$, $X_2$, $X_3$ représentent C et $Z_4$ désigne H, l'un au moins de $Z_1$ et $Z_3$ est différent de H, lorsque $Z_2$ désigne H, Cl, $NO_2$ ou $CH_3$ et $Z_3$ n'est pas H, lorsque $Z_1$ désigne OH et $Z_2$ désigne $OCH_3$;
ainsi que ses sels et complexes métalliques.

**12.** Composé de formule (I) selon la revendication 11, caractérisé par le fait qu'il est choisi parmi l'acide 3,4,5-triméthoxybenzyl iminodiacétique, l'acide 3-hydroxybenzyl iminodiacétique, l'acide N,N'-di(2-méthylbenzyl)éthylènediaminediacétique, l'acide N,N'-di(3-hydroxybenzyl)éthylènediaminediacétique, l'acide N,N'-di(3,4,5-triméthoxybenzyl)éthylènediaminediacétique et son ester méthylique, l'acide N,N'-di(4-hydroxybenzyl)éthylènediaminediacétique, l'acide N,N'-di(3-méthoxybenzyl)éthylènediaminediacétique, l'acide N,N'-di(3,5-diméthoxybenzyl)éthylènediaminediacétique, l'acide N,N'-di(3-hydroxy-4-nitrobenzyl)éthylènediaminediacétique, l'acide N,N'-di(3-chlorobenzyl)éthylènediaminediacétique, l'acide N,N'-di(2,3-diméthyl-4-méthoxybenzyl)éthylènediaminediacétique et l'acide N,N'-di(3-pyridylméthylène)éthylènediaminediacétique, ainsi que leurs sels et complexes métalliques.

**13.** Procédé de préparation du composé de formule (I) selon la revendication 11, caractérisé en ce qu'on réalise les étapes suivantes :

1) on fait réagir l'aldéhyde de formule (II) :

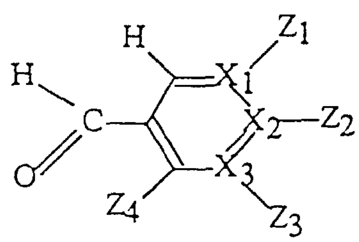

(II)

(A) avec l'ester éthylique de la glycine lorsque $Z_5$ désigne le groupement (a), (B) avec la N-acétyl éthylènediamine lorsque $Z_5$ désigne le groupement (b), ou (C) avec l'éthylènediamine lorsque $Z_5$ désigne le groupement (c),
2) on réduit l'imine ou la diimine correspondante obtenue par du borohydrure de sodium ou par hydrogénation catalytique,
3) on traite l'amine obtenue par le procédé (A) par NaOH pour saponifier l'ester éthylique et la diamine obtenue par le procédé (B) par HCl pour hydrolyser la fonction acétyle,
4) on traite l'amine ou la diamine obtenue en milieu basique par l'acide bromoacétique en présence de monohydrogénocarbonate de sodium, pour obtenir le composé de formule (I) correspondant et on récupère le produit obtenu.

**14.** Utilisation comme antioxydant du composé tel que défini dans l'une quelconque des revendications 1, 11 et 12.

**Patentansprüche**

**1.** Verwendung einer Verbindung der Formel (I)

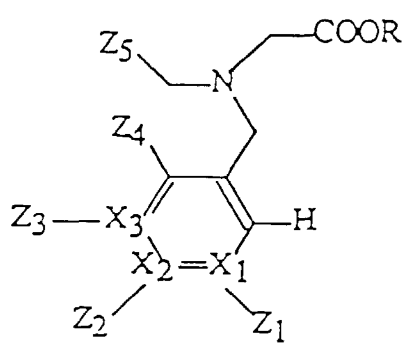

(I),

in der bedeuten:

- $Z_1$, $Z_2$, $Z_3$ unabhängig $NO_2$, COOH, $CF_3$, ein Halogenatom oder eine Gruppe $R_1$, $OR_1$, $SR_1$ oder $NR_1R_2$,

- $Z_4$ eine Gruppe $R_1$,
  wobei R, $R_1$ und $R_2$ unabhängig Wasserstoff oder geradkettiges oder verzweigtes $C_1$-$C_8$-Alkyl bedeuten,
- $X_1$, $X_2$, $X_3$

-C= oder -N=

mit der Maßgabe, daß

wenn $X_1$ = N ist, $X_2$ und $X_3$ = C sind und es keinen Substituenten $Z_1$ an $X_1$ gibt,
wenn $X_2$ = N ist, $X_1$ und $X_3$ = C sind und es keinen Substituenten $Z_2$ an $X_2$ gibt,
wenn $X_3$ = N ist, $X_2$ und $X_1$ gleich C sind und es keinen Substituenten $Z_3$ an $X_3$ gibt,
d.h. es handelt sich um einen Pyridinring, und wenn $X_1$, $X_2$ und $X_3$ jeweils C bedeuten, handelt es sich um einen Benzolring;

- $Z_5$

  die Gruppe

  -COOR (a)

  oder die Gruppe

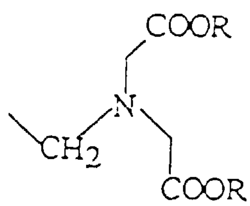

(b)

  oder die Gruppe

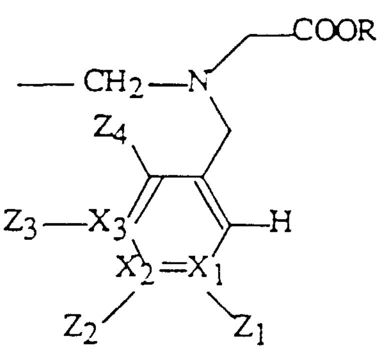

(c),

worin $Z_1$, $Z_2$, $Z_3$, $Z_4$, $X_1$, $X_2$, $X_3$, R, $R_1$ und $R_2$ dasselbe wie weiter oben bedeuten,

sowie ihre Salze und Metallkomplexe.

2. Kosmetische Zusammensetzung, dadurch gekennzeichnet, daß sie mindestens eine wie in Anspruch 1 definierte Verbindung der Formel (I) in einem kosmetisch akzeptablen Medium enthält.

3. Zusammensetzung nach Anspruch 2, dadurch gekennzeichnet, daß sie 0,001 bis 10 Gew.-% mindestens einer

Verbindung der Formel (I), die wie in Anspruch 1 definiert ist, enthält.

**4.** Zusammensetzung, die für die kosmetische Behandlung der Auswirkungen von oxidativem Streß oder den Schutz vor diesen Auswirkungen vorgesehen ist, dadurch gekennzeichnet, daß sie zwei Bestandteile (A) und (B) enthält, wobei

der Bestandteil (A) aus der wie in Anspruch 1 definierten Verbindung der Formel (I) besteht und
der Bestandteil (B) unter Antioxidantien gegen die Fettperoxidation, biologischen Reduktionsmitteln, Mitteln, die Singulettsauerstoff einfangen, Systemen, die Wasserstoffperoxid zu zersetzen vermögen, Systemen für den Schutz vor Superoxidanionen, Systemen, die organische Hydroperoxide zu zersetzen vermögen, entzündungshemmenden Mitteln, UV-Filtern, penetrationsfördernden Mitteln und ihren Kombinationen ausgewählt wird und in der gleichen Zusammensetzung wie der Bestandteil (A) enthalten ist oder dafür vorgesehen ist, getrennt und zeitlich verzögert oder nicht verzögert verwendet zu werden.

**5.** Zusammensetzung nach Anspruch 4, dadurch gekennzeichnet, daß das Antioxidationsmittel gegen die Fettperoxidation unter Vitamin E, Trolox, BHT ausgewählt ist, das biologische Reduktionsmittel unter reduziertem Glutathion und Vitamin C ausgewählt ist, das Mittel, das Singulettsauerstoff einfängt, ein Carotinoid ist, das System, das Wasserstoffperoxid zu zersetzen vermag, ein Catalase- oder Peroxidase-Enzym ist, das System für den Schutz vor Superoxidanionen aus Superoxiddismutasen (SOD), dem Mn-Desferal-Komplex oder dem Diisopropylsalicylat von Kupfer besteht, das System, das organische Hydroperoxide zu zersetzen vermag, die Glutathionperoxidase oder ein System auf Selenbasis ist.

**6.** Verbindung der Formel (I)

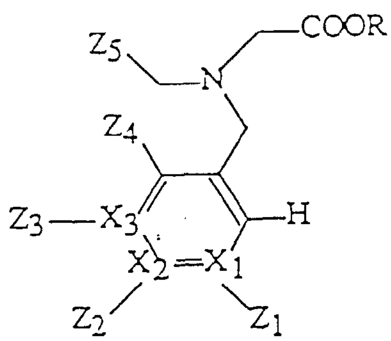

(I),

in der bedeuten:

- $Z_1$, $Z_2$, $Z_3$ unabhängig $NO_2$, COOH, $CF_3$, ein Halogenatom oder eine Gruppe $R_1$, $OR_1$, $SR_1$ oder $NR_1R_2$,
- $Z_4$ eine Gruppe $R_1$,
  wobei R, $R_1$ und $R_2$ unabhängig Wasserstoff oder geradkettiges oder verzweigtes $C_1$-$C_8$-Alkyl bedeuten,
- $X_1$, $X_2$, $X_3$

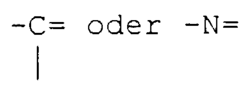

mit der Maßgabe, daß

wenn $X_1$ = N ist, $X_2$ und $X_3$ = C sind und es keinen Substituenten $Z_1$ an $X_1$ gibt,
wenn $X_2$ = N ist, $X_1$ und $X_3$ = C sind und es keinen Substituenten $Z_2$ an $X_2$ gibt,
wenn $X_3$ = N ist, $X_2$ und $X_1$ gleich C sind und es keinen Substituenten $Z_3$ an $X_3$ gibt,
d.h. es handelt sich um einen Pyridinring, und wenn $X_1$, $X_2$ und $X_3$ jeweils C bedeuten, handelt es sich um einen Benzolring;

- $Z_5$

die Gruppe

-COOR (a)

oder die Gruppe

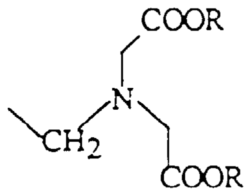

(b)

oder die Gruppe

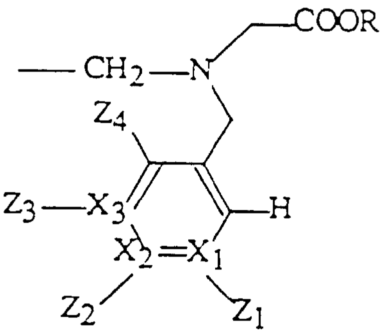

(c) ,

worin $Z_1$, $Z_2$, $Z_3$, $Z_4$, $X_1$, $X_2$, $X_3$, R, $R_1$ und $R_2$ dasselbe wie weiter oben bedeuten,

sowie ihre Salze und Metallkomplexe, zur Verwendung in einem therapeutischen Verfahren zur Behandlung des menschlichen oder tierischen Körpers.

**7.** Verbindung gemäß Formel (I), die wie in Anspruch 6 definiert ist, zur Verwendung für den Schutz des menschlichen Organismus vor oxidativem Streß.

**8.** Pharmazeutische Zusammensetzung, dadurch gekennzeichnet, daß sie in einem pharmazeutisch akzeptablen Medium mindestens eine wie in Anspruch 6 definierte Verbindung der Formel(I) enthält.

**9.** Pharmazeutische Zusammensetzung nach Anspruch 8, dadurch gekennzeichnet, daß sie 0,001 bis 10 Gew.-% mindestens einer wie in Anspruch 6 definierten Verbindung der Formel (I) enthält.

**10.** Zusammensetzung, die für die therapeutische Behandlung der Auswirkungen von oxidativem Streß oder für den Schutz vor den Auswirkungen von oxidativem Streß vorgesehen ist, dadurch gekennzeichnet, daß sie zwei Bestandteile (A) und (B) enthält, wobei

der Bestandteil (A) aus der wie in Anspruch 6 definierten Verbindung der Formel (I) besteht und
der Bestandteil (B) unter Antioxidantien gegen die Fettperoxidation, biologischen Reduktionsmitteln, Mitteln, die Singulettsauerstoff einfangen, Systemen, die Wasserstoffperoxid zu zersetzen vermögen, Systemen für den Schutz vor Superoxidanionen, Systemen, die organische Hydroperoxide zu zersetzen vermögen, entzündungshemmenden Mitteln, UV-Filtern, penetrationsfördernden Mitteln und ihren kombinationen ausgewählt ist und in der gleichen Zusammensetzung wie der Bestandteil (A) enthalten ist oder dafür vorgesehen ist, getrennt und zeitlich verzögert oder nicht verzögert verwendet zu werden.

**11.** Verbindung der Formel (I)

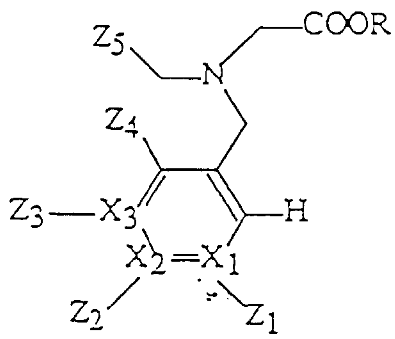

(I),

in der bedeuten:

- $Z_1$, $Z_2$, $Z_3$ unabhängig $NO_2$, COOH, $CF_3$, ein Halogenatom oder eine Gruppe $R_1$, $OR_1$, $SR_1$ oder $NR_1R_2$,
- $Z_4$ eine Gruppe $R_1$,
  wobei R, $R_1$ und $R_2$ unabhängig Wasserstoff oder geradkettiges oder verzweigtes $C_1$-$C_8$-Alkyl bedeuten,
- $X_1$, $X_2$, $X_3$

  $-\underset{|}{C}=$ oder $-N=$

  mit der Maßgabe, daß

  wenn $X_1$ = N ist, $X_2$ und $X_3$ = C sind und es keinen Substituenten $Z_1$ an $X_1$ gibt,
  wenn $X_2$ = N ist, $X_1$ und $X_3$ = C sind und es keinen Substituenten $Z_2$ an $X_2$ gibt,
  wenn $X_3$ = N ist, $X_2$ und $X_1$ gleich C sind und es keinen Substituenten $Z_3$ an $X_3$ gibt,
  d.h. es handelt sich um einen Pyridinring, und wenn $X_1$, $X_2$ und $X_3$ jeweils C bedeuten, handelt es sich um einen Benzolring;

- $Z_5$

  die Gruppe

  -COOR (a)

  oder die Gruppe

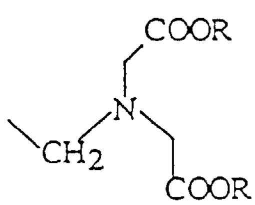

(b)

  oder die Gruppe

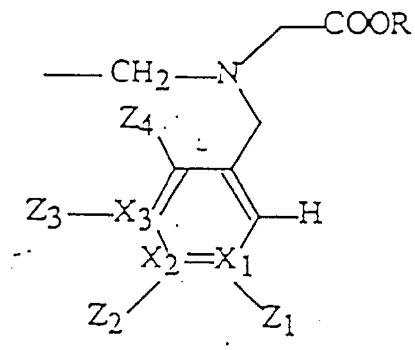

(c),

worin $Z_1$, $Z_2$, $Z_3$, $Z_4$, $X_1$, $X_2$, $X_3$, R, $R_1$ und $R_2$ dasselbe wie weiter oben bedeuten,

wobei die folgenden Verbindungen ausgeschlossen sind: Benzyliminodiessigsäure, 3-Aminobenzyliminodiessigsäure, 3-Nitrobenzyliminodiessigsäure, 4-Nitrobenzyliminodiessigsäure, 4-Aminobenzyliminodiessigsäure, 4-Hydroxybenzyliminodiessigsäure, 4-Dimethylaminobenzyliminodiessigsäure, 4-Methylbenzyliminodiessigsäure, 4-Fluorbenzyliminodiessigsäure, 3-Nitro-4-chlorbenzyliminodiessigsäure, Benzylethylendiamintriessigsäure, 3-Pyridylmethyleniminodiessigsäure, N,N'Bis(3-hydroxy-2,5-dimethyl-4-pyridylmethylen)ethylendiamindiessigsäure
und mit der Maßgabe, daß
wenn $Z_5$ die Gruppe (c), in der $X_1$, $X_2$, $X_3$ C-Atome sind, und $Z_4$ H bedeutet, mindestens einer der Reste $Z_1$ und $Z_3$ verschieden von H ist, wenn $Z_2$ H, Cl, $NO_2$ oder $CH_3$ bedeutet und daß $Z_3$ nicht H ist, wenn $Z_1$ OH und $Z_2$ $OCH_3$ bedeutet.

**12.** Verbindung der Formel (I) nach Anspruch 11, dadurch gekennzeichnet, daß sie ausgewählt ist unter: 3,4,5-Trimethoxybenzyliminodiessigsäure, 3-Hydroxybenzyliminodiessigsäure, N,N'-Di-(2-metehlybenzyl)-ethylendiamindiessigsäuie, N,N'-Di-(3-hydroxybenzyl)-ethylendiamindiessigsäure, N,N'-Di-(3,4,5-Trimethoxybenzyl)-ethylendiamindiessigsäure und ihrem Methylester, N,N'-Di-(4-hydroxybenzyl)-ethylendiamindiessigsäure, N,N'-Di-(3-methoxybenzyl)-ethylendiamindiessigsäure, N,N'-Di-(3,5-dimethoxybenzyl)-ethylendiamindiessigsäure, N,N'-Di-(3-hydroxy-4-nitrobenzyl)-ethylendiamindiessigsäure, N,N'-Di-(3-chlorbenzyl)-ethylendiamindiessigsäure, N,N'-Di-(2,3-dimethyl-4-methoxybenzyl)-ethylendiamindiessigsäure und N,N'-Di-(3-pyridylmethylen)-ethylendiamindiessigsäure sowie ihren Salzen und Metallkomplexen.

**13.** Verfahren zur Herstellung der Verbindung der Formel (I) gemäß Anspruch 11, dadurch gekennzeichnet, daß die folgenden Schritte durchgeführt werden:

1) der Aldehyd der Formel (II)

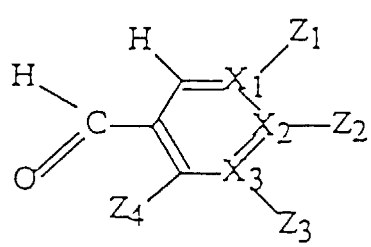

(II)

wird umgesetzt mit

(A) dem Ethylester von Glycin, wenn $Z_5$ die Gruppe (a) bedeutet,
(B) N-Acetylethylendiamin, wenn $Z_5$ die Gruppe (b) bedeutet, oder
(C) Ethylendiamin, wenn $Z_5$ die Gruppe (c) bedeutet,

2) das erhaltene entsprechende Imin oder Diimin wird mit Natriumborhydrid oder durch katalytische Hydrierung reduziert,

3) das durch das Verfahren (A) erhaltene Amin wird zur Verseifung des Ethylesters mit NaOH behandelt, und das durch das Verfahren (B) erhaltene Diamin wird zur Hydrolyse der Acetylgruppe mit HCl behandelt,
4) das erhaltene Amin oder Diamin wird in basischem Medium mit Bromessigsäure in Gegenwart von Natriummonohydrogencarbonat behandelt, um die entsprechende Verbindung der Formel (I) zu erhalten, wonach das erhaltene Produkt isoliert wird.

**14.** Verwendung der wie in einem der Ansprüche 1, 11 und 12 definierten Verbindung als Antioxidationsmittel.

## Claims

**1.** Use of a compound of formula (I):

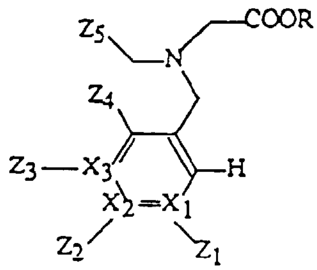

(I)

in which:

$Z_1$, $Z_2$ and $Z_3$, independently of one another, represent $NO_2$, COOH, $CF_3$, a halogen atom or an $R_1$, $OR_1$, $SR_1$ or $NR_1R_2$ group,
$Z_4$ represents an $R_1$ group;
where R, $R_1$ and $R_2$, independently of one another, represent H or a linear or branched $C_1$ to $C_8$ alkyl group,
$X_1$, $X_2$ and $X_3$ represent:

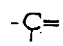 or -N=,

provided that
if $X_1$=N, then $X_2$=$X_3$=C and there is no $Z_1$ substituent at $X_1$,
if $X_2$=N, then $X_1$=$X_3$=C and there is no $Z_2$ substituent at $X_2$,
if $X_3$=N, then $X_2$=$X_1$=C and there is no $Z_3$ substituent at $X_3$,
that is to say that there is a pyridine ring;
if $X_1$, $X_2$ and $X_3$ all three represent C, there is then a benzene ring;
$Z_5$ represents:

the group:

-COOR (a)

or the group:

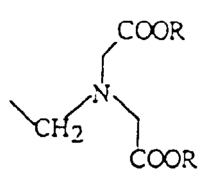

(b)

or the group:

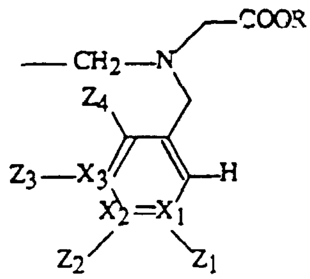

(c)

in which $Z_1$, $Z_2$, $Z_3$, $Z_4$, $X_1$, $X_2$, $X_3$, R, $R_1$ and $R_2$ have the same meanings as above;
or of its salts and metal complexes, for the cosmetic treatment of the body for the purpose of protecting it against oxidative stress.

**2.** Cosmetic composition, characterized in that it contains at least one compound of formula (I) as defined in Claim 1, in a cosmetically acceptable medium.

**3.** Composition according to Claim 2, characterized in that it contains 0.001 to 10% by weight of at least one compound of formula (I) as defined in Claim 1.

**4.** Composition intended for the cosmetic treatment of the effects of oxidative stress or for protecting against these effects, characterized in that it comprises two components (A) and (B):

the component (A) consisting of the compound of formula (I) as defined in Claim 1,
the component (B) being chosen from antilipoperoxidants, biological reducing agents, singlet oxygen scavengers, systems capable of decomposing hydrogen peroxide, systems for protecting against the superoxide anion, systems capable of decomposing organic hydroperoxides, UV screening agents, penetration promoters or their combinations and being present in the same composition as the component (A) or intended to be used separately, optionally separated in time.

**5.** Composition according to Claim 4, characterized in that the antilipoperoxidant agent is chosen from vitamin E, trolox or BHT; the biological reducing agent is chosen from reduced glutathione or vitamin C; the singlet oxygen scavenger is a carotenoid; the system capable of decomposing hydrogen peroxide is a catalase or peroxidase enzyme; the system for protecting against the superoxide anion consists of the SODs, the Mn-desferal complex or copper diisopropylsalicylate; and the system capable of decomposing organic hydroperoxides is glutathione peroxidase or a selenium-based system.

**6.** Compound of formula (I):

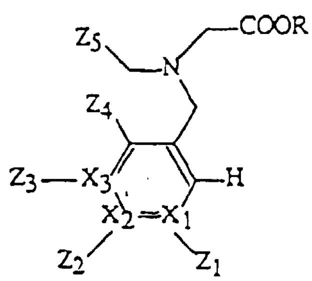

(I)

in which:

$Z_1$, $Z_2$ and $Z_3$, independently of one another, represent $NO_2$, COOH, $CF_3$, a halogen atom or an $R_1$, $OR_1$, $SR_1$ or $NR_1R_2$ group,
$Z_4$ represents an $R_1$ group;

where R, $R_1$ and $R_2$, independently of one another, represent H or a linear or branched $C_1$ to $C_8$ alkyl group, $X_1$, $X_2$ and $X_3$ represent:

$$-\underset{|}{C}= \text{ or } -N=,$$

provided that
if $X_1$=N, then $X_2$=$X_3$=C and there is no $Z_1$ substituent at $X_1$,
if $X_2$=N, then $X_1$=$X_3$=C and there is no $Z_2$ substituent at $X_2$,
if $X_3$=N, then $X_2$=$X_1$=C and there is no $Z_3$ substituent at $X_3$,
that is to say that there is a pyridine ring;
if $X_1$, $X_2$ and $X_3$ all three represent C, there is then a benzene ring;
$Z_5$ represents:

the group:

-COOR (a)

or the group:

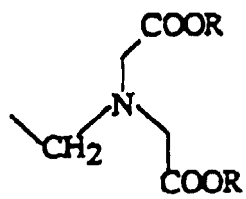
(b)

or the group:

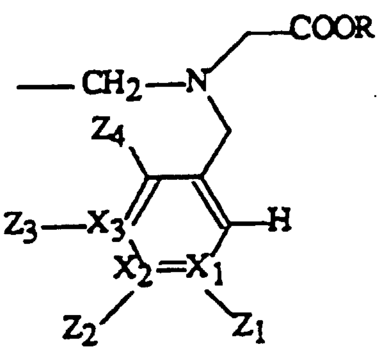
(c)

in which $Z_1$, $Z_2$, $Z_3$, $Z_4$, $X_1$, $X_2$, $X_3$, R, $R_1$ and $R_2$ have the same meanings as above;
or its salts or metal complexes,
for its use in a method for the therapeutic treatment of the human or animal body.

**7.** Compound corresponding to the formula (I) as defined in Claim 6 for its use in the protection of the human body against oxidative stress.

**8.** Pharmaceutical composition, characterized in that it contains, in a pharmaceutically acceptable medium, at least one compound of formula (I) as defined in Claim 6.

**9.** Pharmaceutical composition according to Claim 8, characterized in that it contains 0.001 to 10% by weight of at

least one compound of formula (I) as defined in Claim 6.

**10.** Composition intended for the therapeutic treatment of the effects of oxidative stress or for protecting against these effects, characterized in that it comprises two components (A) and (B):

the component (A) consisting of the compound of formula (I) as defined in Claim 6,
the component (B) being chosen from antilipoperoxidants, biological reducing agents, singlet oxygen scavengers, systems capable of decomposing hydrogen peroxide, systems for protecting against the superoxide anion, systems capable of decomposing organic hydroperoxides, anti-inflammatory agents, penetration promoters or their combinations and being present in the same composition as the component (A) or intended to be used separately, optionally separated in time.

**11.** Compound of formula (I):

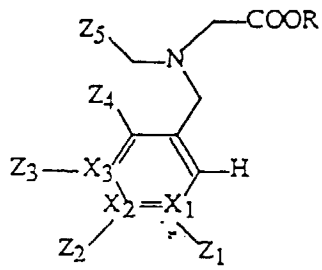

(I)

in which:

$Z_1$, $Z_2$ and $Z_3$, independently of one another, represent $NO_2$, COOH, $CF_3$, a halogen atom or an $R_1$, $OR_1$, $SR_1$ or $NR_1R_2$ group,
$Z_4$ represents an $R_1$ group;
where R, $R_1$ and $R_2$, independently of one another, represent H or a linear or branched $C_1$ to $C_8$ alkyl group,
$X_1$, $X_2$ and $X_3$ represent:

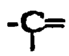 or -N=,

provided that
if $X_1$=N, then $X_2$=$X_3$=C and there is no $Z_1$ substituent at $X_1$,
if $X_2$=N, then $X_1$=$X_3$=C and there is no $Z_2$ substituent at $X_2$,
if $X_3$=N, then $X_2$=$X_1$=C and there is no $Z_3$ substituent at $X_3$,
that is to say that there is a pyridine ring;
if $X_1$, $X_2$ and $X_3$ all three represent C, there is then a benzene ring;
$Z_5$ represents:

the group:

-COOR (a)

or the group:

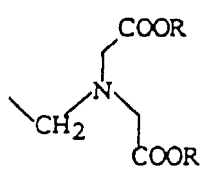

(b)

or the group:

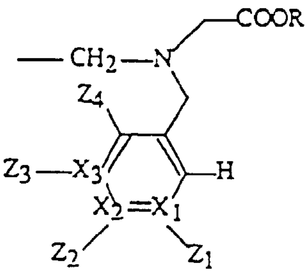

(c)

in which $Z_1$, $Z_2$, $Z_3$, $Z_4$, $X_1$, $X_2$, $X_3$, R, $R_1$ and $R_2$ have the same meanings as above; with the exception of the following compounds: benzyliminodiacetic acid, (3-aminobenzyl)iminodiacetic acid, (3-nitrobenzyl)iminodiacetic acid, (4-nitrobenzyl)iminodiacetic acid, (4-aminobenzyl)iminodiacetic acid, (4-hydroxybenzyl)iminodiacetic acid, (4-dimethylaminobenzyl)iminodiacetic acid, (4-methylbenzyl)iminodiacetic acid, (4-fluorobenzyl)iminodiacetic acid, (3-nitro-4-chlorobenzyl)iminodiacetic acid, benzylethylenediaminetriacetic acid, (3-pyridylmethylene)iminodiacetic [sic] acid and N,N'-bis(3-hydroxy-2,5-dimethyl-4-pyridylmethylene)ethylenediaminediacetic [sic] acid and with the proviso that, when $Z_5$ denotes the group (c) in which $X_1$, $X_2$ and $X_3$ represent C and $Z_4$ denotes H, at least one of $Z_1$ and $Z_3$ is other than H when $Z_2$ denotes H, Cl, $NO_2$ or $CH_3$ and $Z_3$ is not H when $Z_1$ denotes OH and $Z_2$ denotes $OCH_3$;
and its salts and metal complexes.

**12.** Compound of formula (I) according to Claim 11, characterized in that it chosen from (3,4,5-trimethoxybenzyl) iminodiacetic acid, (3-hydroxybenzyl) iminodiacetic acid, N,N'-di(2-methylbenzyl)ethylenediaminediacetic acid, N,N'-di(3-hydroxybenzyl)ethylenediaminediacetic acid, N,N'-di(3,4,5-trimethoxybenzyl)ethylenediaminediacetic acid and its methyl ester, N,N'-di(4-hydroxybenzyl) ethylenediaminediacetic acid, N,N'-di(3-methoxybenzyl)ethylenediaminediacetic acid, N,N'-di(3,5-dimethoxybenzyl)ethylenediaminediacetic acid, N,N'-di-(3-hydroxy-4-nitrobenzyl)ethylenediaminediacetic acid, N,N'-di(3-chlorobenzyl)ethylenediaminediacetic acid, N,N'-di(2,3-dimethyl-4-methoxybenzyl)ethylenediaminediacetic acid, and N,N'-di(3-pyridylmethylene)ethylenediaminediacetic [sic] acid, and their salts and metal complexes.

**13.** Process for the preparation of the compound of formula (I) according to Claim 11, characterized in that the following stages are carried out:

1) the aldehyde of formula (II):

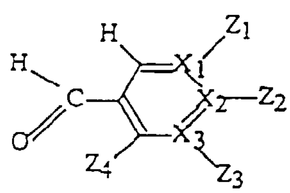

(II)

is reacted (A) with the ethyl ester of glycine when $Z_5$ denotes the group (a), (B) with N-acetylethylenediamine when $Z_5$ denotes the group (b) or (C) with ethylenediamine when $Z_5$ denotes the group (c),
2) the corresponding imine or diimine obtained is reduced with sodium borohydride or by catalytic hydrogenation,
3) the amine obtained by process (A) is treated with NaOH in order to saponify the ethyl ester and the diamine obtained by process (B) is treated with HCl in order to hydrolyse the acetyl functional group,
4) the amine or diamine obtained is treated in basic medium with bromoacetic acid in the presence of sodium monohydrogencarbonate in order to obtain the corresponding compound of formula (I) and the product obtained is recovered.

**14.** Use as antioxidant of the compound as defined in any one of Claims 1, 11 and 12.